# FASCICULE DE BREVET EUROPEEN

(11) **EP 1 150 969 B1**
(45) Date de publication et mention de la délivrance du brevet: **20.11.2002**
(21) Numéro de dépôt: 00901659.3
(22) Date de dépôt: 28.01.2000
(51) Int. Cl.: C07D 401/06, A61K 31/47

(54) **DERIVES DE ALPHA-AZACYCLOMETHYL QUINOLEINE POUR LE TRAITEMENT DE L'INCONTINENCE URINAIRE**
ALPHA-AZACYCLOMETHYLCHINOLINDERIVATE ZUR BEHANDLUNG VON HARNINKONTINENZ
ALPHA-AZACYCLOMETHYL QUINOLINE DERIVATIVES FOR TREATING URINARY INCONTINENCE

(30) Priorité: 02.02.1999 FR 9901145
(43) Date de publication de la demande: 07.11.2001
(73) Titulaire: SANOFI-SYNTHELABO, 75013 Paris (FR)
(72) Inventeur: PHILIPPO, Christophe, F-92500 Rueil Malmaison (FR); BRAUN, Alain, F-92100 Boulogne Billancourt (FR); BOVY, Philippe, R., F-78750 Mareil Marly (FR)
(74) Mandataire: Kugel, Dominique
(86) Numéro de dépôt international: FR0000192
(87) Numéro de publication internationale: WO00046219

(56) Documents cités:
- WO-A-98/33793
- FR-A- 2 756 564

## Description

La présente invention a pour objet des dérivés de α-azacyclométhyl quinoléine, leur préparation et leur application en thérapeutique.

La demande internationale PCT WO 98/33793 décrit des composés répondant à la formule suivante:

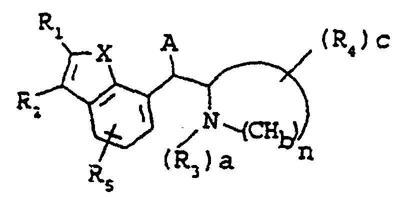

La demande de brevet FR 2 756 564 décrit des composés répondant à la formule suivante:

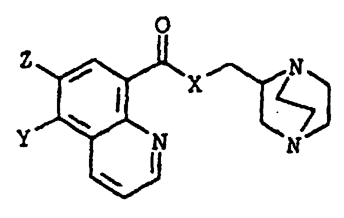

Les composés selon l'invention répondent à la formule générale (I) : 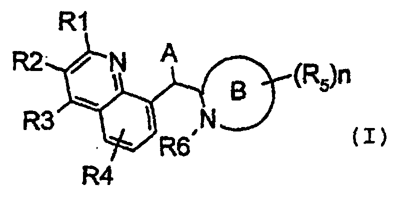 dans laquelle :
A représente soit un atome d'hydrogène, soit un groupe hydroxyle,
B représente une pyrrolidin-2-yl (E) ou 2-pipéridyl (D), 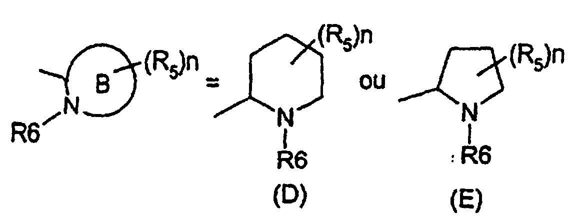
R₁ représente un atome d'hydrogène, un groupe C₁₋₆ alkyle, C₂₋₆ alkényle, C₁₋₂ perfluroalkyle ou C₁₋₆ fluoroalkyle,
   R₂, R₃ ou R₄ représentent chacun, indépendamment l'un de l'autre, un atome d'hydrogène, un groupe C₁₋₆ alkyle ou un groupe C₂₋₆ alkényle, ou
   R₁ et R₂, peuvent encore former ensemble, une chaîne C₁₋₆ alkylène ou une chaîne C₃₋₆ alkénylène,
R₅ représente un groupe C₁₋₆ alkyle,
R₆ représente un atome d'hydrogène, un groupe C₁₋₆ alkyle, un groupe C₂₋₆ alkényle, C₃₋₆ cycloalkyle, C₃₋₆ cycloalkényle ou un benzyle, et
"n" représente 0, 1 ou 2.

Plus particulièrement, les composés pour lesquels A représente groupe hydroxyle sont préférés.

D'autre part les composés pour lesquels R₁ représente un groupe C₁₋₆ alkyle, préférentiellement C₁₋₃ alkyle et plus particulièrement, un éthyle s'avèrent avantageux.

D'autres composés préférés sont les composés pour lesquels R₂ et R3 représentent, indépendamment l'un de l'autre, un atome d'hydrogène ou un groupe C₁₋₃ alkyle, plus particulièrement un méthyle.

Les composés pour lesquels A, R₁, R₂ et R₃ sont tels que définis dans les groupes de composés préférés sont tout particulièrement préférés.

Dans le cadre de la présente invention, on entend par le terme C₁₋₆ alkyle, un groupe aliphatique saturé, linéaire ou ramifié, comprenant de 1 à 6 atomes de carbone, tel que par exemple un groupe méthyle, éthyle, propyle, isopropyle, butyle, isobutyle, *tert*-butyle, etc... Le terme C₁₋₆ alkylène désigne un groupe C₁₋₆ alkyle divalent.

Le terme C₂₋₆ alkényle désigne un groupe aliphatique mono- ou poly-insaturé, linéaire ou ramifié, comprenant de 2 à 6 atomes de carbone. Un groupe alkényle selon l'invention comprend, de préférence, 1 ou 2 insaturations éthyléniques. Le terme C₂₋₆ alkénylène désigne un groupe C₂₋₆ alkényle divalent.

Le terme C₃₋₆ cycloalkyle désigne un système aliphatique saturé cyclique comportant de 3 à 6 atomes de carbone.

Le terme C₃₋₆ cycloalkényle désigne un système aliphatique insaturé cyclique comportant de 3 à 6 atomes de carbone. Selon l'invention, un groupe C₃₋₆ cycloalkényle comprend, de préférence, 1 ou 2 insaturations.

On entend par groupe protecteur Pg, un groupe qui permet d'une part de protéger une fonction réactive telle qu'un hydroxy ou une amine pendant une synthèse et d'autre part de régénérer la fonction réactive intacte en fin de synthèse. Des exemples de groupes protecteurs ainsi que les méthodes de protection et déprotection sont données dans *Protective group in Organic Synthesis* Greene et al., 2nd Ed. (John Wiley & Sons, Inc., New York).

D'autre part, dans les schémas, 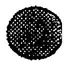 représente un support solide.

Un support solide consiste en un matériau insoluble portant une fonctionalisation destinée à capturer un composé chimique.

Des exemples de tels matériaux sont des polymères, plastiques, résines, polysaccharides et dérivés de la silice. De préférence, les résines sont utilisées et plus préférentiellement, les résines de polystyrène ou de type mixte polystyrène-éthylène glycol (PS-PEG).

La fonctionalisation dépend de la molécule à capturer. Par exemple, celle-ci peut consister en un groupe halogéno, hydroxy, aldéhyde, carboxy, amino, trityl ou thiol.

De tels supports solides comprenant une fonctionalisation adéquate ou qui nécessite une activation préalable par des méthodes connues de l'homme du métier sont disponibles dans le commerce notamment chez Novabiochem, Rapp Polymer, Sigma, Aldrich, Polymer Laboratories ou Argonaut Technologies.

De préférence, le support solide est une résine carboxy activée en chlorure d'acide ou une résine de chlorure de trityle ou une résine de dihydropyrane de Elleman.

Les composés de formule générale (I) peuvent comporter un ou plusieurs atomes de carbone asymétriques. Ils peuvent donc exister sous forme d'énantiomères ou de diastéréoisomères. Ces enantiomères, diastéréoisomères, ainsi que leurs mélanges, y compris les mélanges racémiques font partie de l'invention.

Les composés de formule générale (I) peuvent se présenter sous forme de base libre ou de sels d'addition à des acides, qui font également partie de l'invention. Ces sels, selon la présente invention, comprennent ceux avec des acides minéraux ou organiques qui permettent une séparation ou une cristallisation convenable des composés de formule (I), tels que l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le citrate, le pamoate, le 2-naphtalènesulfonate, le paratoluènesulfonate. Mêmes si les sels pharmaceutiquement acceptables sont préférés, les autres sels font partis de la présente invention. Ces sels peuvent être préparés, selon des méthodes connues de l'homme du métier, par exemple, par réaction du composé de formule (I) sous forme de base avec l'acide dans un solvant approprié, tel qu'une solution alcoolique ou un solvant organique, puis séparation du milieu qui le contient par évaporation du solvant ou par filtration.

Les composés dérivés d'α-azacyclométhylquinoléine de formule (I) selon l'invention, peuvent être préparés selon différents procédés. Les composés de formule (I), en particulier ceux pour lesquels A représente un groupe hydroxyle, peuvent être préparés selon le procédé décrit dans le schéma 1.

Selon ce procédé, les composés de formule (I) pour lesquels R₆ représente un atome d'hydrogène sont obtenus par clivage du support solide des composés de formule (II) provenant de la déprotection du groupe protecteur Pg de l'azote du composé de formule (III), selon des méthodes connues de l'homme du métier. Un tel groupe protecteur peut être par exemple un groupe C₁₋₆ alcoxycarbonyle tel que le *tert*-butyloxycarbonyle (tBoc) . Les significations de R₁, R₂, R₃, R₄, R₅ et n des composés de formule (II) et (III), sont celles indiquées dans la formule (I) .

L'aminé secondaire des composés de formule (II) ainsi obtenue peut alors être fonctionnalisée, selon des procédés connus de l'homme du métier, par exemple par amination réductrice (selon les conditions décrites par Balasubranian et al Tetrahedron letters, vol37, n°27, pp 4819-4822) pour donner, après clivage du support solide, un composé de formule (I) dans laquelle R₆ n'est pas un hydrogène.

Les composés de formule (III) peuvent être obtenus par capture à l'aide d'un support solide activé du mélange réactionnel provenant de la réaction d'un aldéhyde de formule (VIII), dans laquelle Pg représente un groupe protecteur, connu de l'homme du métier, avec un dérivé (IV) obtenu par métallation du dérivé halogéné de formule (V). Les significations de R₁, R₂, R₃, R₄, R₅, B et n des composés de formule (V), (IV) et (VIII), sont celles indiquées dans la formule (I). La réaction de métallation peut être réalisée dans un solvant organique tel que le tétrahydrofurane par formation du réactif de Grignard du dérivé halogéné de formule (V), dans laquelle Y représente un halogène, ou plus avantageusement par réaction du dérivé halogéné de formule (V) en présence de n-butyllithium de préférence à des températures de l'ordre de -78°C. Le terme "métal" dans la formule (IV) représente, par exemple, du lithium Li.

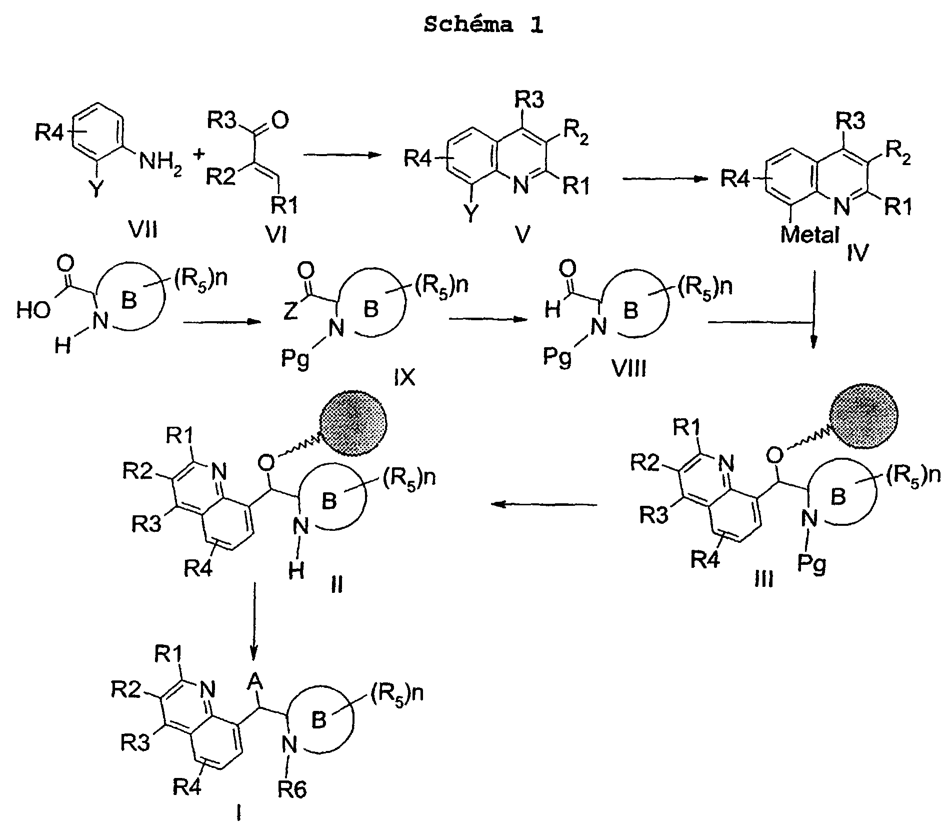

Les aldéhydes de formule (VIII) peuvent être obtenus par réduction, par exemple selon la méthode décrite par Jurczack J. et al., Chem. Rev (1989), 89, 149, à partir des dérivés d'α-aminoacides *N*-protégés correspondants de formule (IX) provenant eux-mêmes des α-aminoacides de formule (X). R₅, B et n ont les significations indiquées dans la formule (I). Pg est défini tel que précédemment et Z représente un groupe, connu de l'homme du métier, permettant une réduction contrôlée qui s'arrête à la formation d'un aldéhyde, entre autres, la *N*-méthyl-*O*-méthyl-hydroxamine (Nahm et Weinreb (1981), Tet. Lett., 22, 3815)

Les composés de formule (V) peuvent être eux-mêmes préparés soit par une réaction de Skraup ou de Doebner-Miller. Selon ce procédé et dans les conditions définies par Belser P. Tetrahedron **1996**, Vol 52,N°8, 2937-2944 ou avantageusement dans les conditions définies par Z. Song J. Heterocyclic Chem. **1993**, 30, 17-21, une aniline de formule (VII), pour laquelle Y représente un halogène tel que un brome, un iode, et un aldéhyde ou une cétone α,β-insaturés formule (VI) sont chauffés en présence d'un agent déhydrant tel que l'acide sulfurique et d'un oxydant tel que l'iodure de sodium pour former un dérivé quinoléine substituée en position 8 par Y. Les significations de R₁, R₂, R₃ et R₄ des composés de formule (V), (VI) et (VII) sont celles indiquées dans la formule (I).

Alternativement, les composés de formule (I), dans laquelle A représente un groupe hydroxyle, peuvent être préparés sans utiliser la résine en gardant le groupe hydroxyle libre.

Les composés de formule (I) selon l'invention, pour lesquels A est un atome d'hydrogène, peuvent être préparés par déshydroxylation d'un composé de formule (I) correspondant, où A est un groupe hydroxyle.

La réaction de déshydroxylation peut être effectuée, de manière connue de l'homme du métier, par réaction avec du triéthylsilane et de l'acide trifluroacétique ou selon le procédé décrit par Myers, A. G.; Movassaghi, M.; Zheng, B J. Am. Chem. Soc. 1997, 119, 8572-8573.

Les composés de départ, notamment ceux de formule VII, VI, sont disponibles dans le commerce ou peuvent être préparés selon des méthodes connues de l'homme du métier.

Les exemples qui suivent illustrent les procédés et techniques appropriés pour la préparation de cette invention, sans toutefois limiter l'étendue de la revendication. Les spectres RMN et IR, les spectres de masse, les analyses de pureté par HPLC (purifications par chromatographie liquide couplée à un détecteur de masse) confirment les structures des composés.

### Exemple 1 : (R,R)-2-[(2,3-diméthylquinol-8-yl)hydroxyméthyl]-1-éthyl-pyrrolidine et (R,S)-2-[(2,3-diméthylquinol-8-yl)hydroxyméthyl]-1-éthyl-pyrrolidine

### (1) 2,3-diméthyl-8-bromoquinoléine

Dans un tricol de 500 ml, muni d'un thermomètre et d'un pousse-seringue, on introduit 55 ml d'acide sulfurique à 70%, 19 g (110,4 mmoles) de 2-bromoaniline et 0,22 g (1,47 mmole) d'iodure de sodium. Le mélange est chauffé à 110°C. On additionne, à la vitesse de 4,23 ml/h, la 2-méthylbutén-2-al (17 ml ; 176 mmoles) et on poursuit l'agitation pendant 40 min. Le mélange réactionnel, refroidi à température ambiante, est versé sur de la glace pilée (21). Le mélange obtenu est rendu basique par l'ajout de carbonate de sodium puis on procède à une extraction par le dichlorométhane (3x300 ml). Les phases organiques sont rassemblées, séchées sur sulfate de magnésium et concentrées. Le résidu est purifié par colonne chromatographique sur silice (solvant d'élution cyclohexane : acétate d'éthyle 6:4).

On obtient 8,34g (Rendement : 32 %) de 2,3-diméthyl-8-bromoquinoléine - F:78°C

### (2) (R)-2-[(Méthoxyméthylamino)carbonyl]pyrrolidine-1-carboxylate de 1,1-diméthyléthyle.

Dans un tricol de 500 ml, muni d'un thermomètre et d'une ampoule d'addition de 250 ml, on place 13,0 g (56,8 mmoles) de (*R*)-2-carboxypyrrolidine-1-carboxylate de 1,1-diméthyléthyle et 190 ml de dichlorométhane. Le mélange est refroidi à -20°C par un bain de carboglace, et on ajoute 7,91 ml (57 mmoles) de triéthylamine suivi de 7,01 ml (56,8 mmoles) de chlorure de pivaloyle. Le mélange est agité vigoureusement pendant 15 minutes à -10°C, puis on laisse-la température remonter à 0°C, et on ajoute, goutte à goutte, une solution de 11,07 g (113 mmoles) de chlorhydrate de *N,O*-diméthylhydroxamine et de 17,4 ml (125 mmoles) de triéthylamine dans 160 ml de dichlorométhane. On laisse le mélange réactionnel revenir à température ambiante en 120 minutes puis on le refroidit à 0°C par un bain de glace et on ajoute 100 ml d'une solution 0,1 N d'acide chlorhydrique. La phase organique est lavée successivement par 100 ml d'une solution de soude 0,2 M, et par 200 ml de saumure, puis séchée sur sulfate de magnésium et concentrée. Le résidu est purifié par chromatographie sur colonne de silice (solvant d'élution : 50% d'acétate d'éthyle dans le cyclohexane). On obtient 14,26 g (Rendement : 92 %) de (*R*)-2-[(méthoxyméthylamino)carbonyl]pyrrolidine-1-carboxylate de 1,1-diméthyléthyle sous forme d'une huile incolore.

### (3) (R)-2-Formylpyrrolidine-1-carboxylate de 1,1-diméthyléthyle.

Dans un ballon de 125 ml, on place 0,80 g (21 mmoles) d'hydrure d'aluminium lithium et 25 ml d'éther éthylique. La suspension est refroidie à -10°C par un bain de glace et on ajoute, goutte à goutte, une solution de 4,57 g (16,8 mmoles) de (*R*)-2-[(méthoxyméthylamino)carbonyl]pyrrolidine-1-carboxylate de 1,1-diméthyléthyle dans 25 ml d'éther éthylique. Le mélange réactionnel est agité pendant 45 minutes à -10°C, puis on ajoute une solution de 3,43 g (25,2 mmoles) de bisulfate de potassium dans 50 ml d'eau. On procède à une extraction par l'acétate d'éthyle (3 x 80 ml). Les phases organiques sont rassemblées, lavées par 120 ml d'une solution d'acide chlorhydrique molaire, 120 ml d'une solution saturée de bicarbonate de sodium, 120 ml d'eau, 120 ml de saumure, séchées sur sulfate de magnésium et concentrées. On obtient 3,03 g (Rendement : 84,5 %) de (*R*)-2-formylpyrrolidine-1-carboxylate de 1,1-diméthyléthyle sous forme d'une huile incolore.

### (4) Carboxylate greffé sur une résine 1% divinylbenzène-polystyrène de (R,R)-2-[(2,3-diméthylquinol-8-yl)hydroxyméthyl]pyrrolidine-1-carboxylate de 1,1-diméthyléthyle et de (R,S)-2-[(2,3-diméthylquinol-8-yl)hydroxyméthyl]pyrrolidine-1-carboxylate de 1,1-diméthyléthyle.

Dans un tricol de 100 ml, on place 0,7 g (3 mmoles) de 2,3-diméthyl-8-bromoquinoléine et 20 ml de tétrahydrofurane anhydre. La solution est refroidie à -78°C par un bain de carboglace dans l'acétone, et on ajoute, goutte à goutte, 1,875 ml (3 mmoles) d'une solution de n-butyl lithium 1,6 M dans l'hexane puis, à la suite, une solution de 0,4 g (2 mmoles) de (*R*)-2-formylpyrrolidine-1-carboxylate de 1,1-diméthyléthyle dans 5 ml de tétrahydrofurane anhydre. Le mélange réactionnel est agité pendant 75 minutes à -78°C puis on ajoute, goutte à goutte 10 ml d'eau. On procède à une extraction par l'acétate d'éthyle (3 x 40 ml). Les phases organiques sont rassemblées, lavées par 60 ml d'eau, 60 ml de saumure, séchées sur sulfate de magnésium et concentrées. Le résidu est placé dans un tube en verre épais et on ajoute 5 ml de dichlorométhane, 0,50 g de chlorure de carboxyl greffé sur une résine 1% divinylbenzène-polystyrène (préparée selon le procédé décrit par Panek et al Tetrahedron letters, vol. 37, n°45, 1996, pp 8151-8154), 0,061 g de DMAP (diméthylaminopyridine)et 0,57 ml de triéthylamine. Le tube est fermé par un bouchon en téflon, placé dans un bain à ultrason à 50° pendant 16 h. La résine est filtrée, rincée par du dichlorométhane (10x10 ml),et séchée sous vide (550 mg obtenu) pour donner le carboxylate greffé sur une résine 1% divinylbenzène-polystyrène de (*R,R*)-2- [(2,3-diméthylquinol-8-yl)hydroxyméthyl] pyrrolidine-1-carboxylate de 1,1-diméthyléthyle et de (*R,S*)*-*2-[(2,3-diméthylquinol-8-yl)hydroxyméthyl]pyrrolidine-1-carboxylate de 1,1-diméthyléthyle.

Une analyse élémentaire permet de déterminer un pourcentage d'azote de 3,15% soit une résine chargée à 1,1 mmole/g.

### (5) Carboxylate greffé sur une résine 1% divinylbenzène-polystyrène de (R,R)-2-[(2,3-diméthylquinol-8-yl)hydroxyméthyl]pyrrolidine et de (R,S)-2-[(2,3-diméthylquinol-8-yl)hydroxyméthyl]pyrrolidine.

Dans un flacon en PTFE, on place 0,10 g de carboxylate greffé sur une résine 1% divinylbenzène-polystyrène de (*R,R*)-2-[(2,3-diméthylquinol-8-yl)hydroxyméthyl]pyrrolidine-1-carboxylate de 1,1-diméthyléthyle et de (*R,S*)-2-[(2,3-diméthylquinol-8-yl)hydroxyméthyl]pyrrolidine-1-carboxylate de 1,1-diméthyléthyle et 5 ml d'une solution d'acide trifluoroacétique (TFA) dans le dichlorométhane (9/1). La suspension est agitée 4 h à température ambiante. La résine est filtrée, rincée par du dichlorométhane (5x5 ml), une solution méthanol/tétrahydrofurane (THF) (1/2) (5x5 ml) puis séchée sous vide pour donner le carboxylate greffé sur une résine 1% divinylbenzène-polystyrène de *(R,R*)-2-[(2,3-diméthylquinol-8-yl)hydroxyméthyl]pyrrolidine et de (*R,S*)-2-[(2,3-diméthylquinol-8-yl)hydroxyméthyl]pyrrolidine.

### (6) Carboxylate greffé sur une résine 1% divinylbenzène-polystyrène de (R,R)-2-[(2,3-diméthylquinol-8-yl)hydroxyméthyl]-1-éthyl-pyrrolidine et de (R,S)-2-[(2,3-diméthylquinol-8-yl)hydroxyméthyl]-1-éthyl-pyrrolidine.

Dans un tube, on place 0.1 g de carboxylate greffé sur une résine 1% divinylbenzène-polystyrène de (*R,R*)-2-[(2,3-diméthylquinol-8-yl)hydroxyméthyl]pyrrolidine et de (*R,S*)-2-[(2,3-diméthylquinol-8-yl)hydroxyméthyl]pyrrolidine, 5 ml d'un mélange éthanol/N-N-diméthylformamide (DMF) (1/3), 0,4 ml d'acétaldéhyde et 0,4 ml de complexe borane-pyridine (BAP). Le mélange est agité pendant 36 h à température ambiante. La résine est filtrée, rincée par un mélange éthanol/DMF (1/3)(3x5 ml), du dichlorométhane (2x5 ml), du méthanol (2x5 ml), du dichlorométhane (2x5 ml), puis séchée sous vide pour donner le carboxylate greffé sur une résine 1% divinylbenzène-polystyrène de (*R,R*)-2-[(2,3-diméthylquinol-8-yl)hydroxyméthyl]-1-éthyl-pyrrolidine et de (*R,S*)-2-[(2,3-diméthylquinol-8-yl)hydroxyméthyl]-1-éthyl-pyrrolidine.

### (7) (R,R)-2-[(2,3-diméthylquinol-8-yl)hydroxyméthyl]-1-éthyl-pyrrolidine et (R,S)-2-[(2,3-diméthylquinol-8-yl)hydroxyméthyl]-1-éthyl-pyrrolidine.

Dans un tube en verre épais, on place 0.10 g de Carboxylate greffé sur une résine 1% divinylbenzène-polystyrène de (R,R)-2-[(2,3-diméthylquinol-8-yl)hydroxyméthyl]-1-éthyl-pyrrolidine et de (*R,S*)-2-[(2,3-diméthylquinol-8-yl)hydroxyméthyl]-1-éthyl-pyrrolidine, 5 ml de THF et 2,5 ml d'hydroxyde de tétrabutylammonium en solution molaire dans le méthanol. Le tube est fermé par un bouchon en téflon, placé dans un bain à ultrason à 50° pendant 16 h. La résine est filtrée, rincée par de une solution de THF/méthanol (2/1) (5x10 ml). La solution provenant des lavages est concentrée sous vide et on ajoute 15 ml d'acétate d'éthyle. Cette solution est lavée à l'eau (3x10 ml), séchée sur sulfate de magnésium et concentrée sous vide pour donner un mélange de (*R,R*)-2-[(2,3-diméthylquinol-8-yl)hydroxyméthyl]-1-éthyl-pyrrolidine et de (*R,S*)-2-[(2,3-diméthylquinol-8-yl)hydroxyméthyl]-1-éthyl-pyrrolidine.

### Exemple 2 : 2-Ethyl-3-méthyl-8-bromoquinoléine

Dans un tricol de 1000 ml, muni d'un thermomètre et d'un pousse-seringue, on introduit 240 ml d'acide sulfurique à 70%, 91,4 g (531 mmoles) de 2-bromoaniline et 0,77 g (5.14 mmole) d'iodure de sodium. Le mélange est chauffé à 170°C. On additionne, à la vitesse de 25 ml/h, la 2-méthylpentèn-2-al (100 ml ; 876 mmoles) et on poursuit l'agitation pendant 1 h. Le mélange réactionnel, refroidi à température ambiante, est versé sur de la glace pilée (21). Le mélange obtenu est rendu basique par l'ajout de carbonate de sodium puis on procède à une extraction par le dichlorométhane (3x300 ml). Les phases organiques sont rassemblées, séchées sur sulfate de magnésium et concentrées. Le résidu est purifié par colonne chromatographique sur silice (solvant d'élution cyclohexane : acétate d'éthyle 6:4)puis repris avec un peu de pentane et trituré à froid. Le précipité obtenu est filtré et rincé avec un minimum de pentane.

On obtient 11,2 g (Rendement : 8,4 %) de 2-éthyl-3-méthyl-8-bromo-quinoléine sous forme d'une poudre blanche - F:54°C

En reproduisant essentiellement le même procédé de l'exemple 1, avec les produits de départ adéquats, on a préparé d'autres composés de formule (I) conforme à l'invention, répertoriés dans le tableau ci-après.

Le tableau suivant rassemble les composés de l'invention, ainsi que leurs caractéristiques physiques.

### TABLEAUX

- Dans ces tableaux :: - 2HC1 représente un dichlorhydrate,
- nPr représente un groupe propyle linéaire,
- i-Pr représente un groupe iso-propyle,
- Et représente un groupe éthyle,
- Me représente un groupe méthyle.
- i-Bu représente un groupe iso-butyle,
- Bn représente un groupe benzyle,

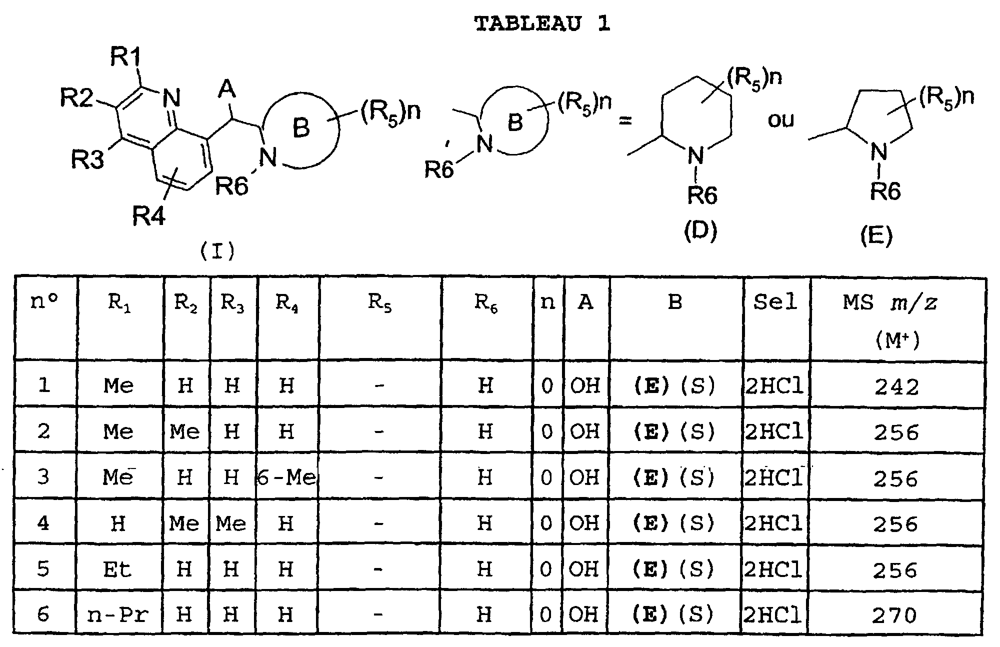

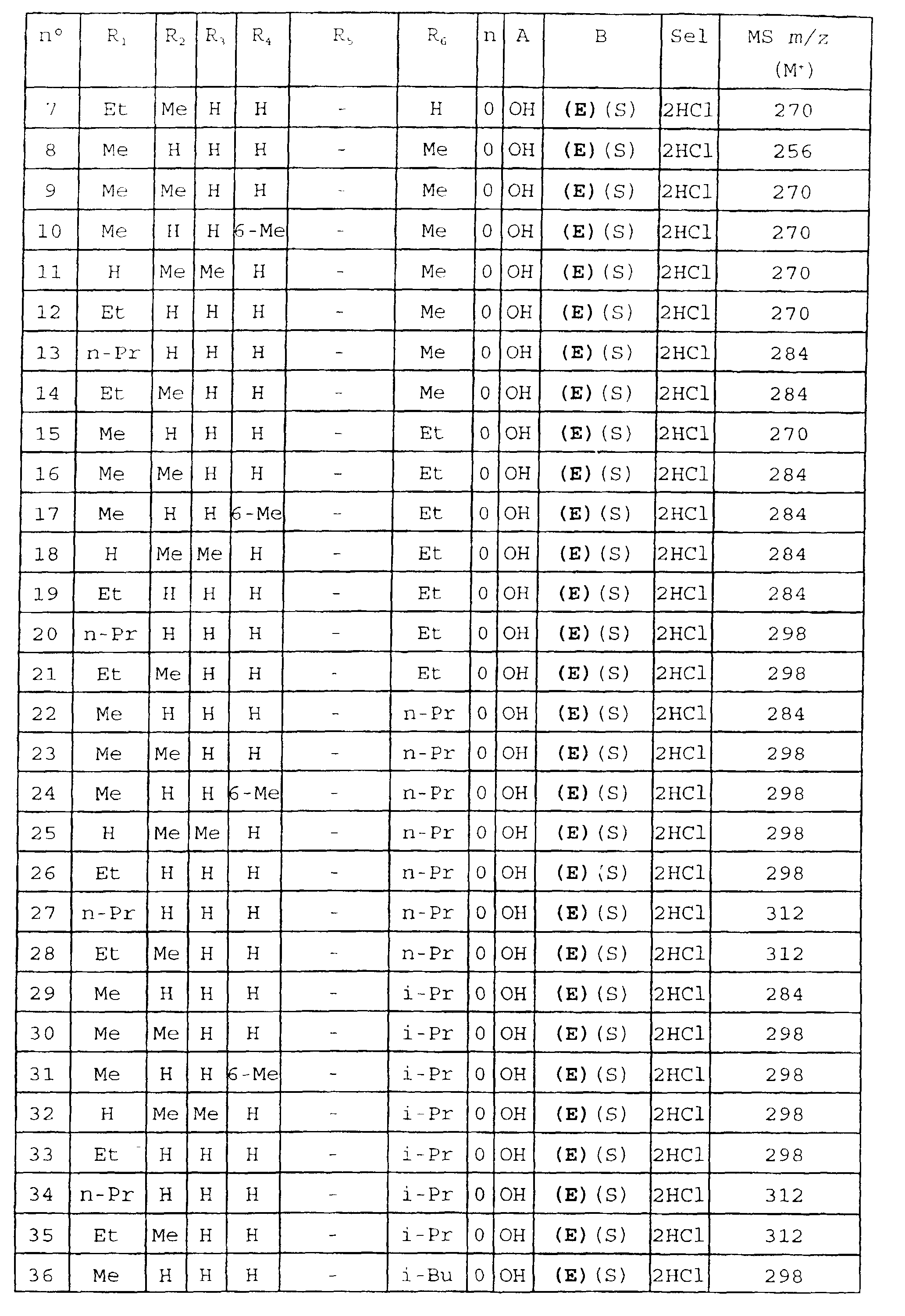

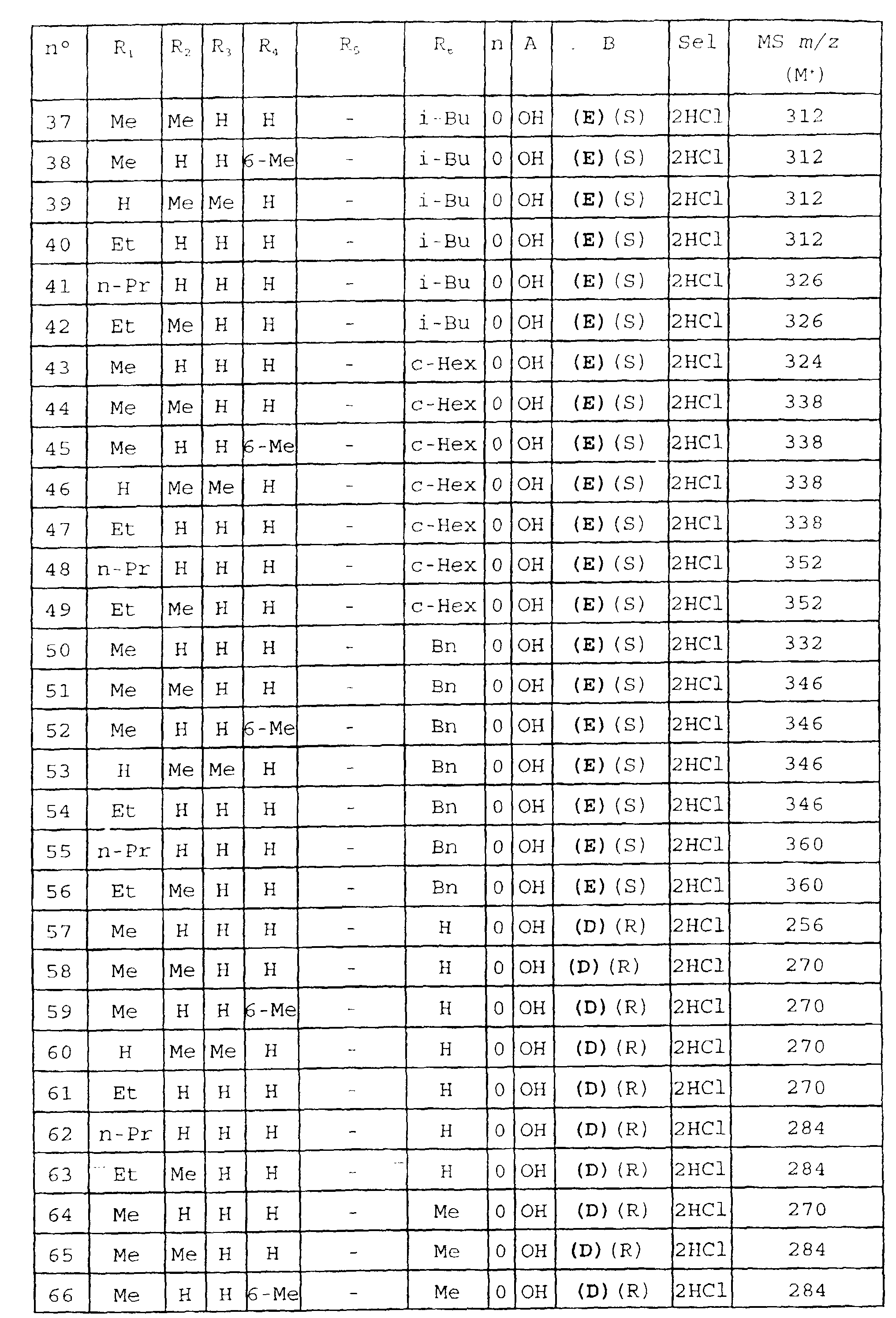

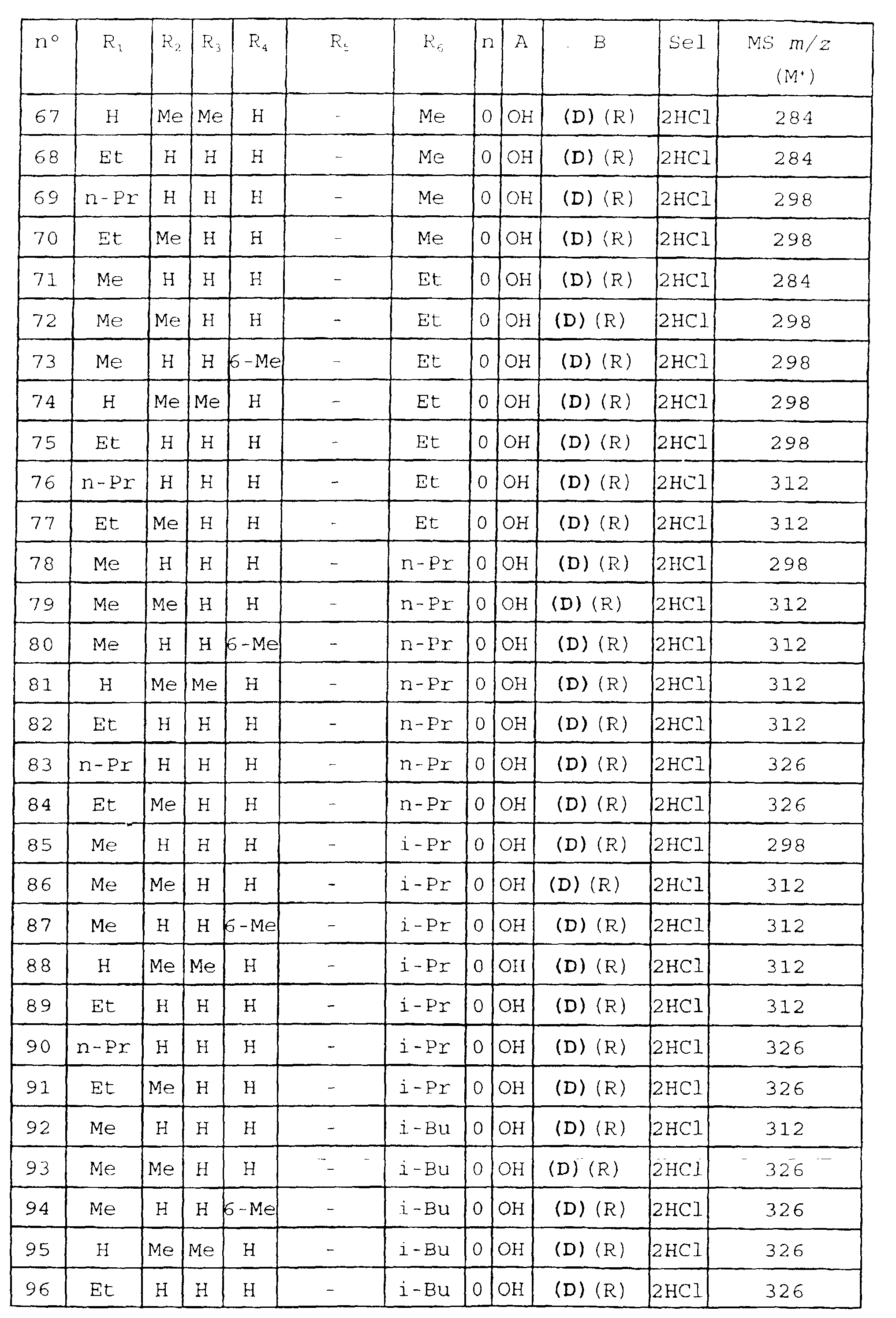

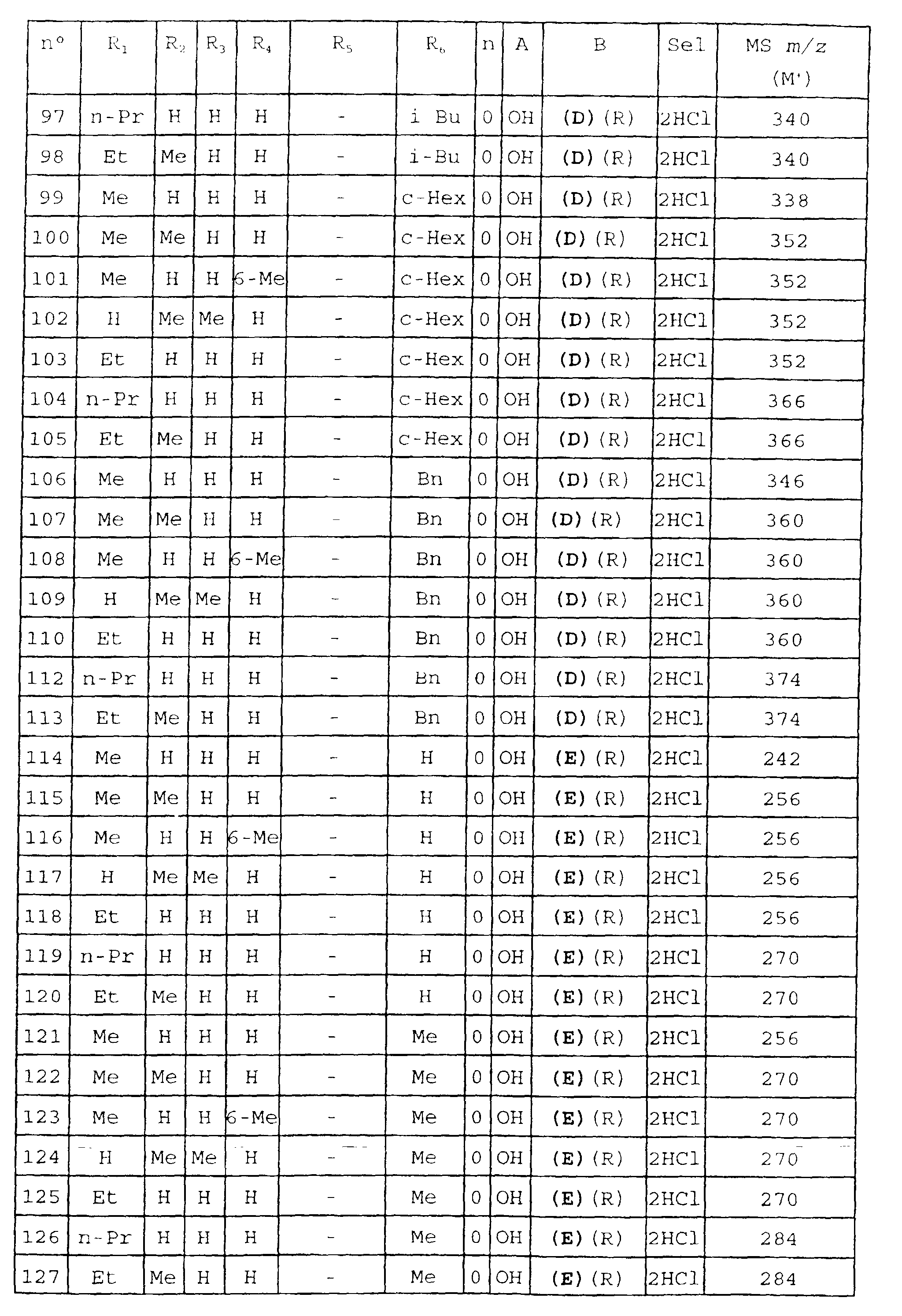

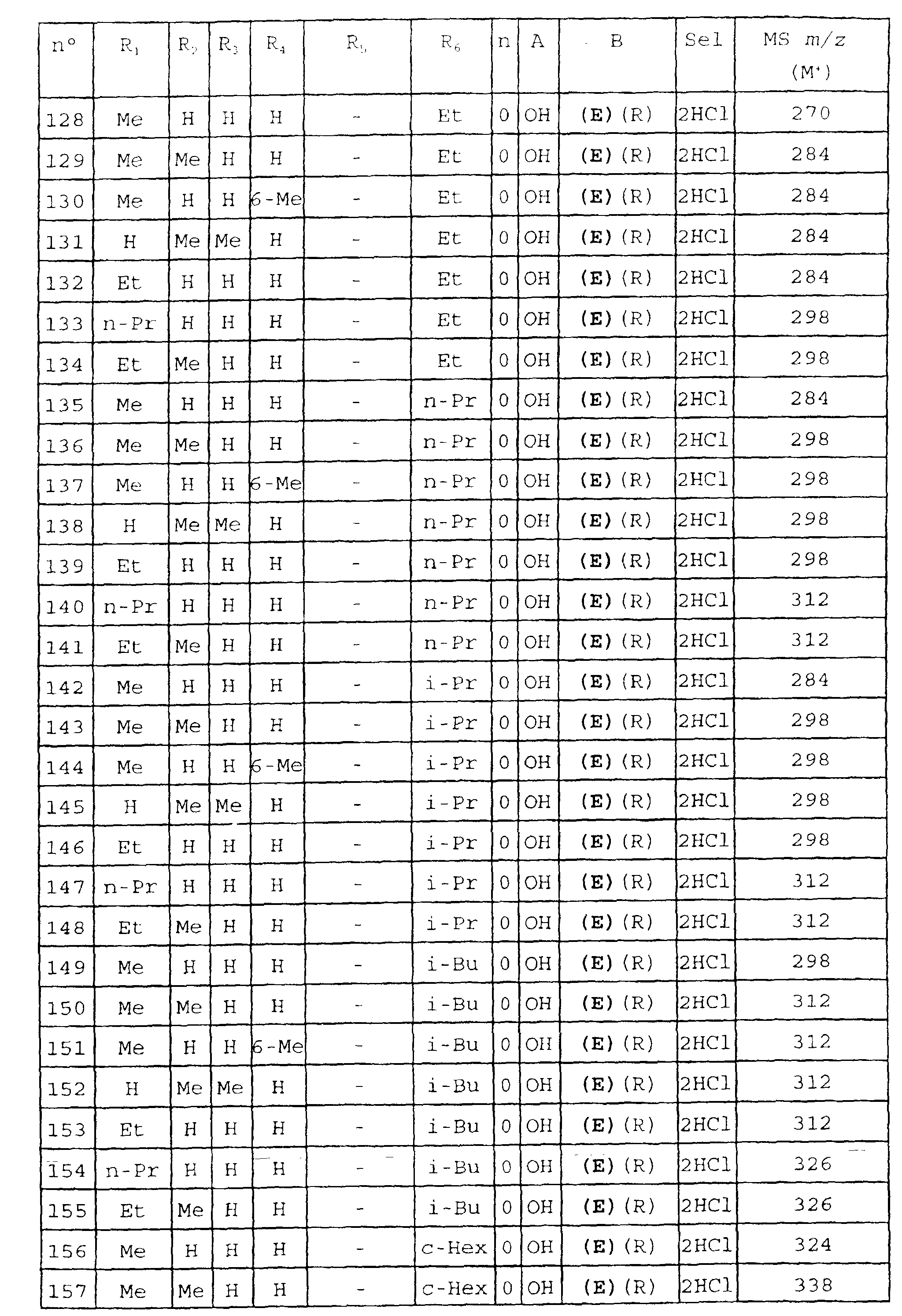

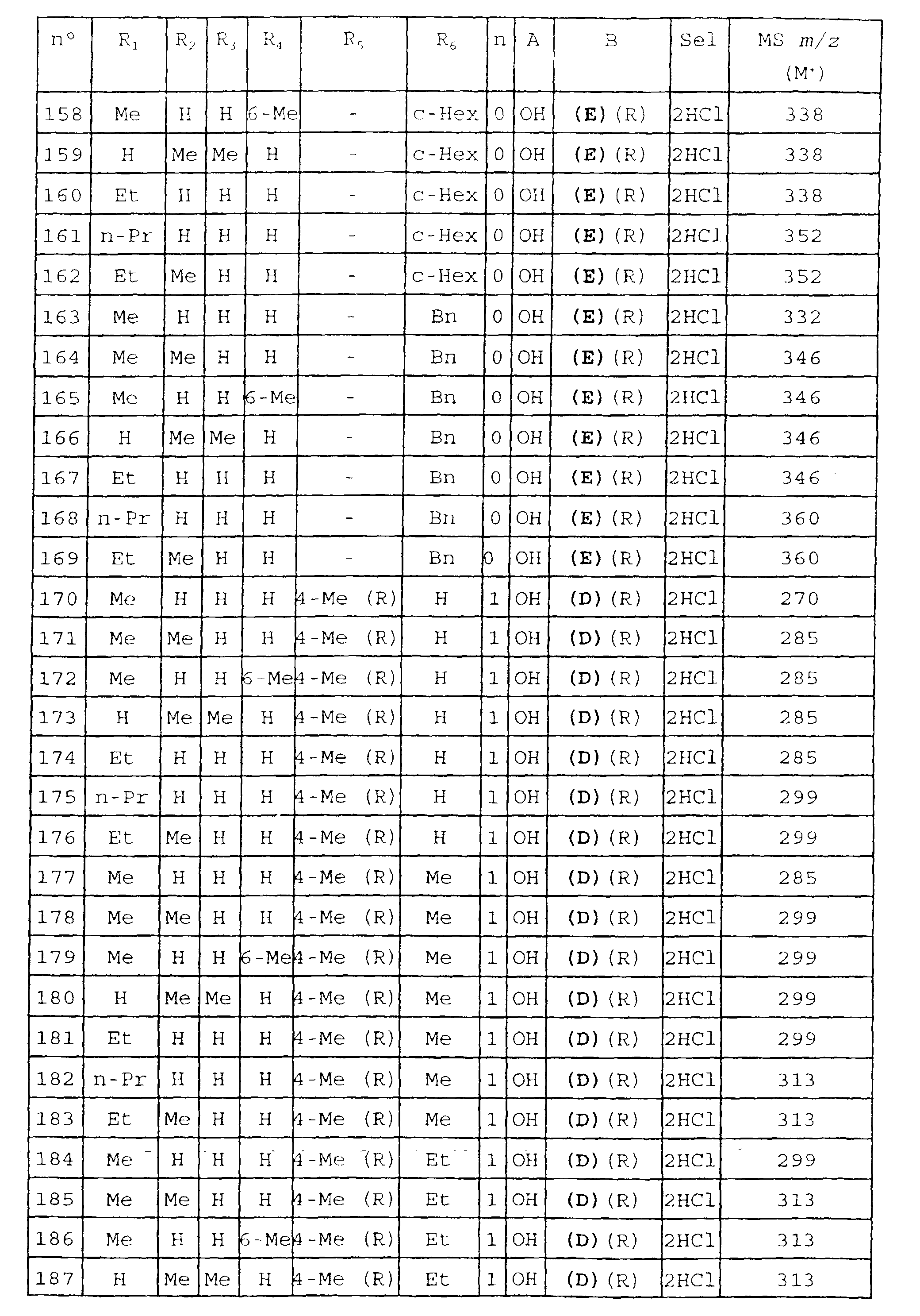

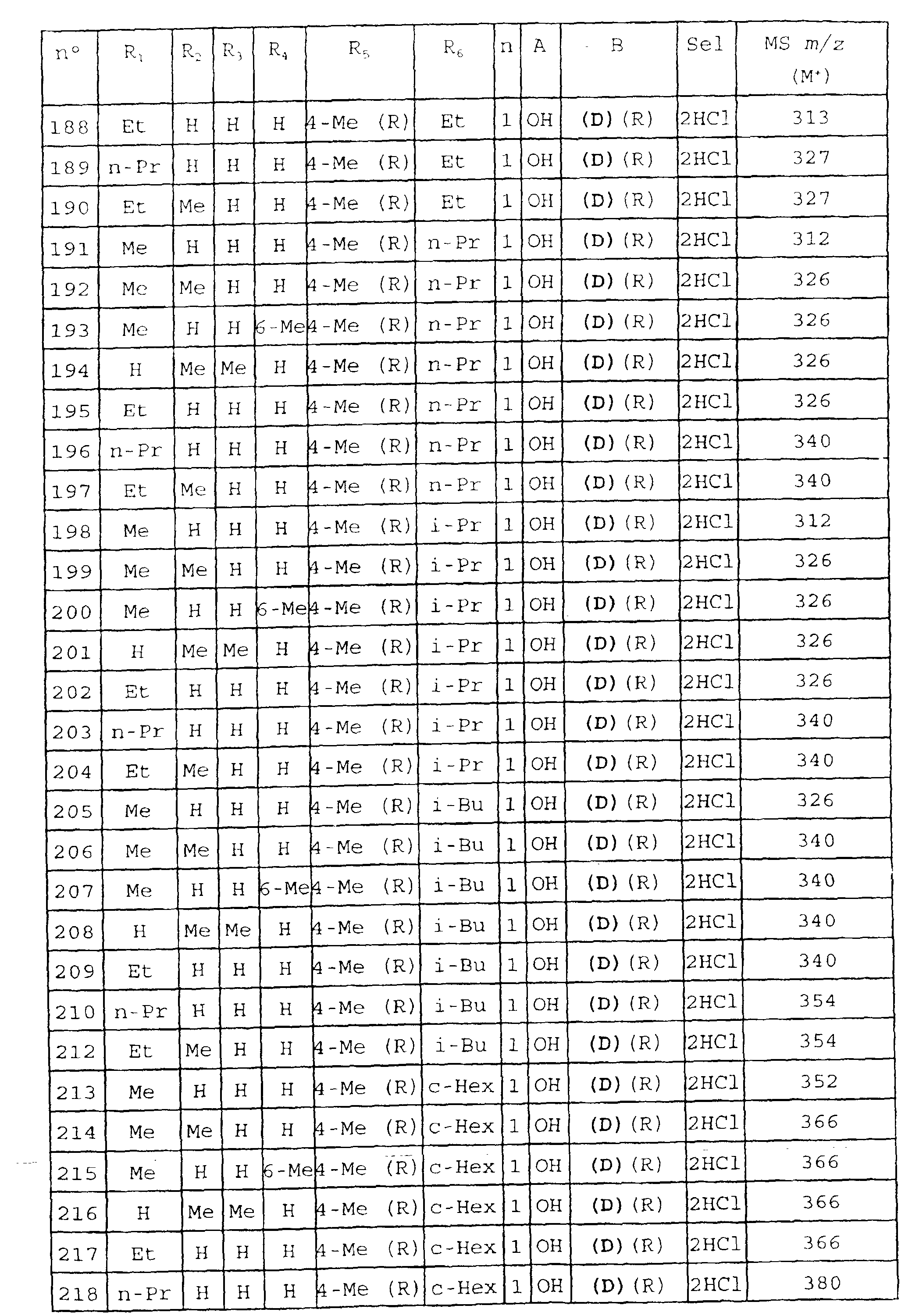

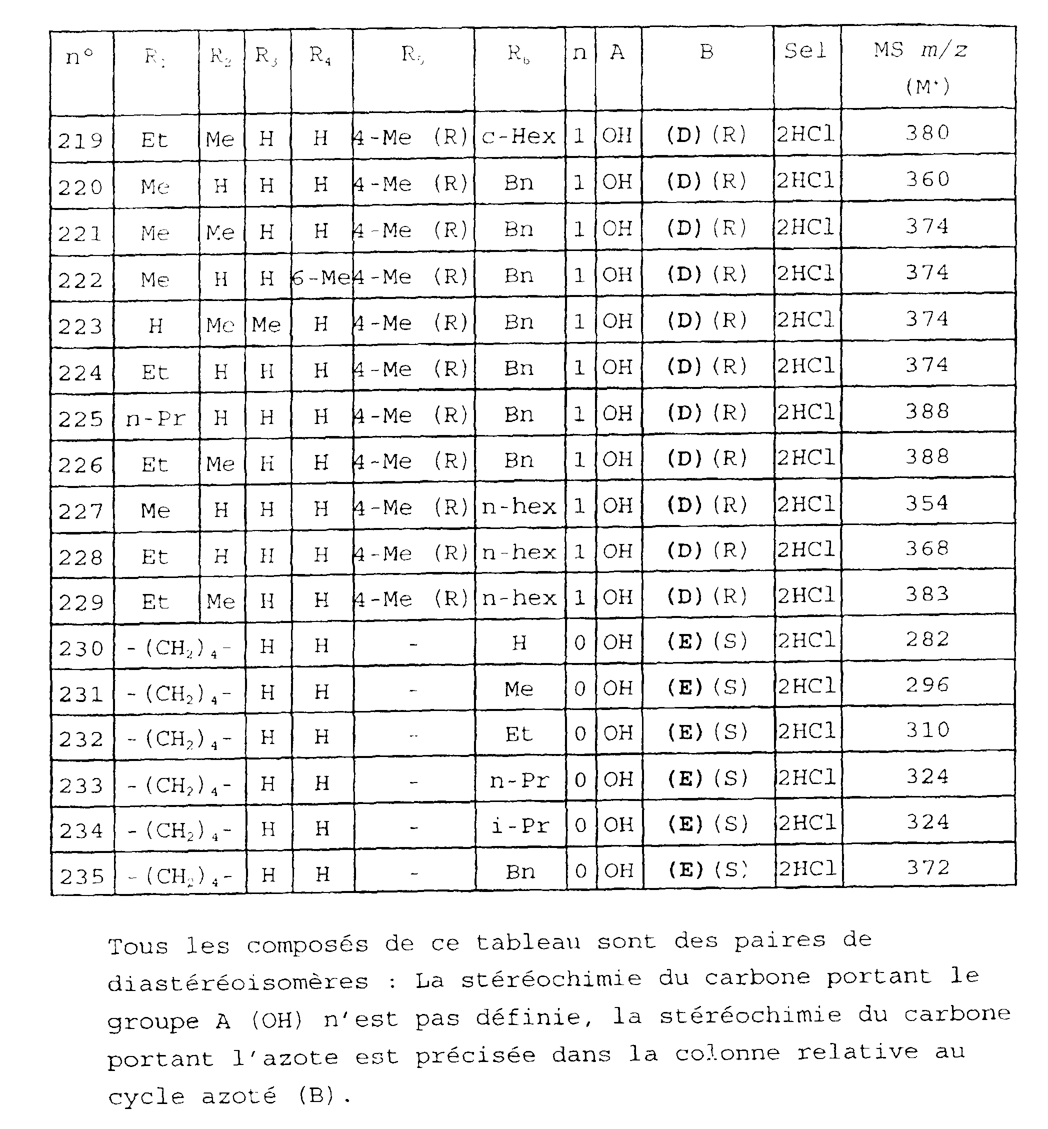

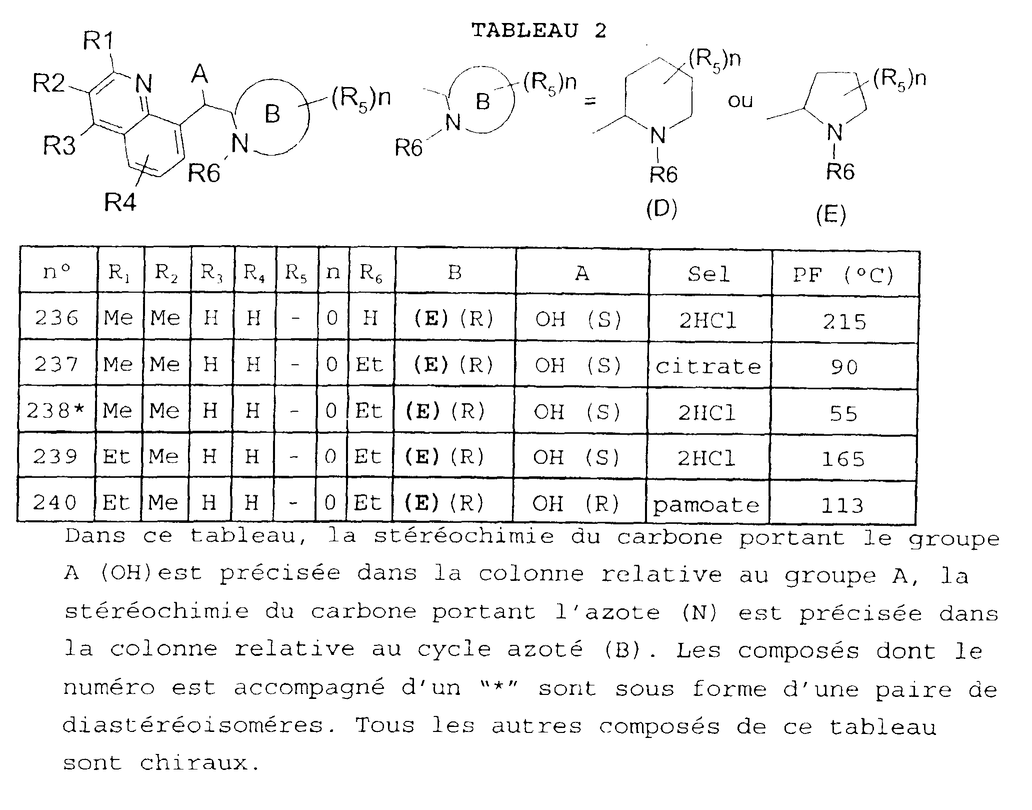

Les composés de l'invention ont été soumis à des tests biologiques destinés à mettre en évidence leur activité contractile sur les muscles lisses urétraux et artériels.
1. L'activité in *vitro* des composés de l'invention a été étudiée sur les muscles lisses urétraux et artériels. Ces essais ont été réalisés sur des lapins femelles néo-zélandais pesant de 3 à 3,5 kg. Les animaux ont été tués par dislocation vertébrale, puis on a prélevé des anneaux de tissu d'artères mésentériques et d'urètre. Ces anneaux de tissu ont été immergés dans une solution de Krebs modifiée, oxygénée par un mélange de 95 % de 0₂ et 5 % de CO₂. Chaque échantillon de tissu a été soumis à une tension de 1 g puis on a introduit de la phényléphrine à des doses cumulatives et établi la courbe dose/réponse. Après rinçage des échantillons, on a introduit le composé à étudier à des doses cumulatives et établi la courbe dose/réponse. L'effet contractile de chaque composé est évalué par le calcul du pD₂ (logarithme négatif de la concentration d'agoniste qui induit 50% de la contraction maximale) ainsi que par l'effet maximum représentant le pourcentage de la contraction maximum obtenue avec la phényléphrine (% Eₘₐₓ).
   Les résultats obtenus montrent que les composés conformes à l'invention, présentent :
   * un pD₂ urètre, habituellement compris entre 4 et 8
   * un pD₂ artère habituellement inférieur à 3,
   * un %Eₘₐₓ urètre supérieur à 30, habituellement compris entre 40 et 90,
   * un %Eₘₐₓ artère habituellement égal à zéro.
2. L'activité *in* vitro des composés de l'invention a été étudiée sur les veines saphènes de micro-porc Yucatan. Le tissu est découpé en hélice et est monté dans un cuve à organes isolés dans une solution de Krebs modifiée oxygénée par un mélange de 95% O₂ et 5% CO₂ maintenue à 37°C. Le vaisseau est relié à un capteur isométrique sous une tension basale de 1 g et est connecté à un polygraphe permettant l'enregistrement des variations tensionnelles. La viabilité de chaque préparation est testée par pré-stimulation avec la noradrénaline 3µM. Après rinçage, le composé à étudier est introduit et sa courbe concentration-réponse construite de façon cumulative jusqu'à l'obtention d'une réponse maximale. L'effet contractile de chaque composé est évalué par calcul de la CE₅₀ (concentration produisant 50% de la réponse maximale).
   Les composés de l'invention ont permis l'obtention d'une activité veinoconstrictrice avec une valeur de CE₅₀ habituellement comprise entre 1 µM et 100 µM.
   Les composés de l'invention peuvent être utilisés dans le traitement de l'insuffisance veineuse et de l'ulcère veineux.
3. L'activité *in vivo* des composés de l'invention sur la pression sanguine et urétrale a été étudiée chez le rat amyélé et le lapin, selon les protocoles suivants :

### * Rats démédulés

Les rats Wistar sont anesthésiés et démédulés (suivant la technique décrite par Gillespie, MacLaren A. and Polock D., A method of stimulating different segments of the autonomic outflow from the spinal column to various organs in the pithed cat and rat ; Br. J. Pharmacol., 1970, 40 : 257-267).

Les cathéters sont introduits par l'aorte et une veine jugulaire. Un autre cathéter est introduit dans l'urètre par une incision exécutée dans la vessie. Les composés à tester sont administrés à des doses croissantes par infusion intraveineuse.

Les résultats sont exprimés en doses (µg/kg) nécessaires pour augmenter la pression urétrale de 10 cm d'eau (PU₁₀) ou la pression artérielle de 10 mm de Hg (PA₁₀), soit 10/760 atm, ou de 50 mm de Hg (PA₅₀), soit 50/760 atm.

Les composés de l'invention ainsi testés, ont permis l'obtention :
- d'une PU₁₀ avec des doses inférieures à 500 µg/kg, habituellement comprises entre 50 et 200 µg/kg,
- d'une PA₁₀ avec des doses supérieures à 600 µg/kg, habituellement comprises entre 600 et 2000 µg/kg,
- la PA₅₀ n'a pu être atteinte.

L'ensemble des résultats ci-dessus, montrent que les composés de l'invention ont une forte action contractile urétrale et une faible action contractile artérielle.

Ils peuvent être utilisés comme médicament, en particulier en tant qu'agent contractant des muscles lisses, et plus particulièrement encore, dans le traitement de l'incontinence urinaire d'effort. Dans cette indication, les composés selon l'invention présentent une bonne efficacité et, habituellement, des effets secondaires moindres que les médicaments conventionnellement utilisés pour un tel traitement, notamment pour ce qui concerne les effets secondaires affectant le système cardio-vasculaire, en particulier les lits artériels.

Les composés selon l'invention peuvent aussi être mis en oeuvre pour le traitement des insuffisances veineuses, de la migraine, des troubles gastro-intestinaux et en tant que vaso-constricteur de la muqueuse nasale.

L'utilisation des composés selon l'invention pour la préparation d'un médicament destiné à traiter les pathologies ci-dessus mentionnées fait partie intégrante de l'invention.

Selon un autre de ses aspects, la présente invention concerne des compositions pharmaceutiques renfermant en tant que principe actif, un composé selon l'invention.

Ainsi, ces compositions pharmaceutiques contiennent une dose efficace d'un composé selon l'invention ou d'un sel ou hydrate pharmaceutiquement acceptable de celui-ci, et un ou plusieurs excipients pharmaceutiques convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique ou rectale, le principe actif de formule (I) ci-dessus son sel ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif peut varier entre 0,1 et 50 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 1 à 500 mg, de principe actif en combinaison avec un excipient pharmaceutique. Cette dose unitaire peut être administrée 1 à 5 fois par jour de façon à administrer un dosage journalier de 0,5 à 5000 mg, de préférence de 1 à 2500 mg.

Par exemple, lorsqu'on prépare une composition solide sous forme de comprimés, on mélange l'ingrédient actif principal avec un excipient pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières. Les comprimés peuvent être réalisés par différentes techniques, compression directe, granulation sèche, granulation humide ou fusion à chaud.

Selon un deuxième exemple, on obtient une préparation en gélules en mélangeant l'ingrédient actif avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

La présente invention selon un autre de ses aspects, concerne également une méthode de traitement des pathologies ci-dessus indiquées qui comprend l'administration d'un composé selon l'invention ou un des ses sels ou hydrates.

## Revendications

1. Composé de formule (I) 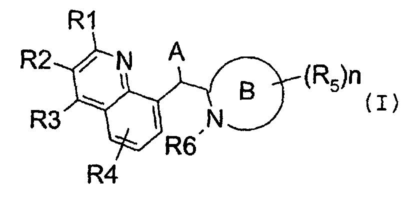 dans laquelle :
A représente soit un atome d'hydrogène, soit un groupe hydroxyle,
B représente une pyrrolidin-2-yl (E) ou 2-pipéridyl (D), 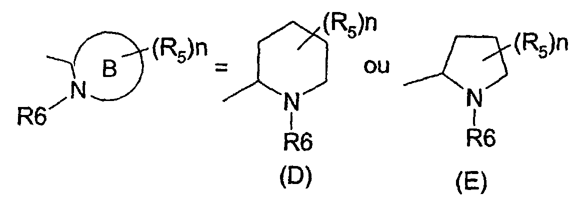
R₁ représente un atome d'hydrogène, un groupe C₁₋₆ alkyle, C₂₋₆ alkényle, C₁₋₂ perfluroalkyle ou C₁₋₆ fluoroalkyle,
R₂, R₃ ou R₄ représentent chacun, indépendamment l'un de l'autre, un atome d'hydrogène, un groupe C₁₋₆ alkyle ou un groupe C₂₋₆ alkényle, ou
R₁ et R₂, peuvent encore former ensemble, une chaîne C₁₋₆ alkylène ou une chaîne C₃₋₆ alkénylène,
R₅ représente un groupe C₁₋₆ alkyle,
R₆ représente un atome d'hydrogène, un groupe C₁₋₆ alkyle, un groupe C₂₋₆ alkényle, C₃₋₆ cycloalkyle, C₃₋₆ cycloalkényle ou un benzyle, et
"n" représente 0, 1 ou 2, et éventuellement et ses sels.

2. Composé selon la revendication 1, **caractérisé en ce que** A représente un groupe hydroxyle.

3. Composé selon la revendication 1 ou 2, **caractérisé en ce que** R₁ représente C₁₋₃ alkyle.

4. Composé selon la revendication 1, 2 ou 3, **caractérisé en ce que** R₂ et R₃ représentent, indépendamment l'un de l'autre, un atome d'hydrogène ou un groupe C₁₋₃ alkyle.

5. Composé selon la revendication 1 **caractérisé en ce qu'**il consiste en le (*R,R*)-2-[(2,3-diméthylquinol-8-yl)hydroxyméthyl]-1-éthyl-pyrrolidine ou le (*R,S*)-2-[(2,3-diméthylquinol-8-yl)hydroxyméthyl]-1-éthyl-pyrrolidine.

6. , Procédé de préparation d'un composé de formule (I) selon la revendication 1, **caractérisé en ce que** l'on fait réagir un aldéhyde de formule (VIII) 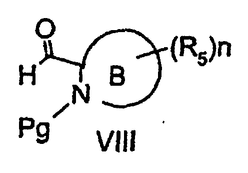 avec un dérivé organométallique de quinoléine de formule (IV) 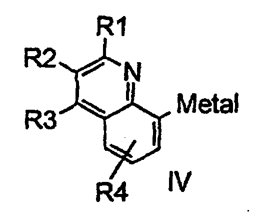 dans lesquelles les significations de R₁, R₂, R₃, R₄, R₅, B et n de l'aldéhyde de formule (VIII) et du dérivé quinoléine de formule (IV) sont celles définies pour le composé de formule (I) selon la revendication 1 et Pg représente un groupe protecteur, suivie d'une déprotection de l'amine, pour donner un composé de formule (I), selcn la revendication 1 dans laquelle A représente un hydroxy et R₆ représente un hydrogène, et éventuellement d'une fonctionalisation de l'amine pour donner un composé de formule (I) selon la revendication 1, dans laquelle R₆ est différent d'un hydrogène, et éventuellement suivi d'une déshydroxylation pour donner un composé de formule (I) dans lequel A est un hydrogène.

7. Médicament **caractérisé en ce qu'**il est constitué d'un composé selon la revendication 1, 2, 3 ou 4.

8. Composition pharmaceutique **caractérisée en ce qu'**elle comprend au moins un composé, selon de la revendication 1, 2, 3 ou 4, et un ou plusieurs excipients appropriés.

## Patentansprüche

1. Verbindung der Formel (I) 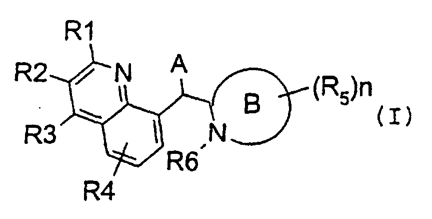 in der:
A entweder ein Wasserstoffatom oder eine Hydroxylgruppe darstellt,
B Pyrrolidin-2-yl (E) oder 2-Piperidyl (D) bedeutet 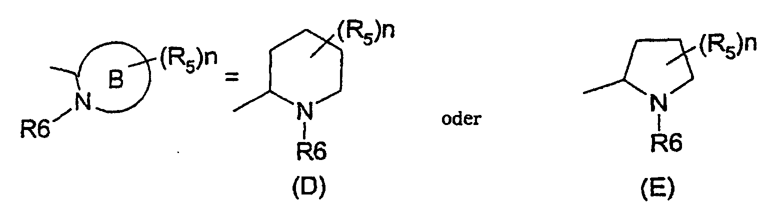
R₁ ein Wasserstoffatom, eine C₁₋₆-Alkylgruppe, C₂₋₆-Alkenylgruppe, C₁₋₂-Perfluoralkylgruppe oder C₁₋₆-Fluoralkylgruppe darstellt,
R₂, R₃ oder R₄ jeweils unabhängig voneinander Wasserstoffatome, C₁₋₆-Alkylgruppen oder C₂₋₆-Alkenylgruppen darstellen oder
R₁ und R₂ gemeinsam eine C₁₋₆-Alkylenkette oder eine C₃₋₆-Alkenylenkette bilden,
R₅ eine C₁₋₆-Alkylgruppe darstellt,
R₆ ein Wasserstoffatom, eine C₁₋₆-Alkylgruppe, C₂₋₆-Alkenylgruppe, C₃₋₆-Cycloalkylgruppe, C₃₋₆-Cycloalkenylgruppe oder eine Benzylgruppe darstellt und
"n" 0, 1 oder 2 bedeutet, und gegebenenfalls deren Salze.

2. Verbindung nach Anspruch 1, **dadurch gekennzeichnet**, daB A eine Hydroxylgruppe bedeutet.

3. Verbindung nach Anspruch 1 oder 2, **dadurch gekennzeichnet, daß** R₁ C₁₋₃-Alkyl bedeutet.

4. Verbindung nach Anspruch 1, 2 oder 3, **dadurch gekennzeichnet, daß** R₂ und R₃ unabhängig voneinander Wasserstoffatome oder C₁₋₃-Alkylgruppen bedeuten.

5. Verbindung nach Anspruch 1, nämlich (R,R)-2-[(2,3-Dimethylchinol-8-yl)-hydroxymethyl)-1-ethyl-pyrrolidin oder (*R,S*)-2-[(2,3-Dimethylchinol-8-yl)-hydroxymethyl]-1-ethyl-pyrrolidin.

6. Verfahren zur Herstellung einer Verbindung der Formel (I) nach Anspruch 1, **dadurch gekennzeichnet, daß** man einen Aldehyd der Formel (VIII) 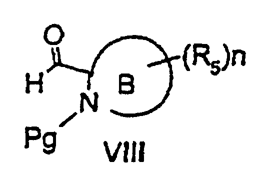 mit einem metallorganischen Chinolinderivat der Formel (IV) 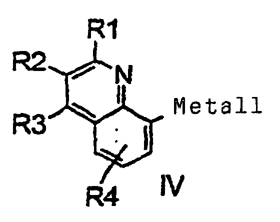 worin R₁, R₂, R₃, R₄, R₅, B und n des Aldehyds der Formel (VIII) und des Chinolinderivats der Formel (IV) die für die Verbindung der Formel (I) in Anspruch I angegebenen Bedeutungen besitzen und Pg eine Schutzgruppe darstellt, umsetzt, gefolgt von der Abspaltung der Schutzgruppe des Amins zur Bildung einer Verbindung der Formel (I) nach Anspruch 1, worin A eine Hydroxygruppe und R₆ ein Wasserstoffatom bedeuten, und gegebenenfalls Funktionalisierung des Amins zur Bildung einer Verbindung der Formel (I) nach Anspruch 1, worin R₆ von Wasserstoff verschieden ist, und gegebenenfalls gefolgt von einer Deshydroxylierung zur Bildung einer Verbindung der Formel (I). worin A Wasserstoff bedeutet.

7. Arzneimittel, **dadurch gekennzeichnet, daß** es aus einer Verbindung nach Anspruch 1, 2, 3 oder 4 gebildet ist.

8. Pharmazeutische Zubereitung, **dadurch gekennzeichnet, daß** sie mindestens eine Verbindung nach den Ansprüchen 1, 2, 3 oder 4 und ein oder mehrere geeignete Trägermaterialien umfaßt.

## Claims

1. Compound of formula (I) 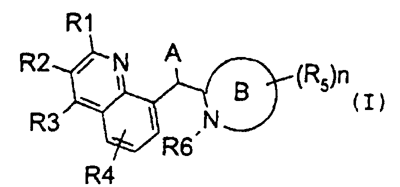 in which:
A represents either a hydrogen atom or a hydroxyl group,
B represents a 2-pyrrolidinyl (E) or 2-piperidyl (D), 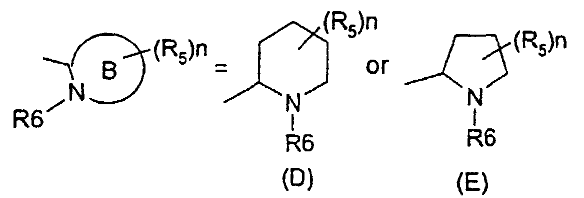
R₁ represents a hydrogen atom, a C₁₋₆ alkyl, C₂₋₆ alkenyl, C₁₋₂ perfluoroalkyl or C₁₋₆ fluoroalkyl group,
R₂, R₃ or R₄ represent, independently of each other, a hydrogen atom, a C₁₋₆ alkyl group or a C₂₋₆ alkenyl group, or
R₁ and R₂ together may also form a C₁₋₆ alkylene chain or a C₃₋₆ alkenylene chain,
R₅ represents a C₁₋₆ alkyl group,
R₆ represents a hydrogen atom, a C₁₋₆ alkyl group, a C₂₋₆ alkenyl group, a C₃₋₆ cycloalkyl group, a C₃₋₆ cycloalkenyl group or a benzyl, and
"n" represents 0, 1 or 2, and optionally the salts thereof.

2. Compound according to Claim 1, **characterized in that** A represents a hydroxyl group.

3. Compound according to Claim 1 or 2, **characterized in that** R₁ represents C₁₋₃ alkyl.

4. Compound according to Claim 1, 2 or 3, **characterized in that** R₂ and R₃ represent, independently of each other, a hydrogen atom or a C₁₋₃ alkyl group.

5. Compound according to Claim 1, **characterized in that** it consists of (R,R)-2-[(2,3-dimethylquinol-8-yl)hydroxymethyl]-1-ethylpyrrolidine or (R,S)-2-[(2,3-dimethylquinol-8-yl)hydroxymethyl]-1-ethylpyrrolidine.

6. Process for preparing a compound of formula (I) according to Claim 1, **characterized in that** an aldehyde of formula (VIII) 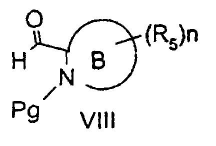 is reacted with an organometallic quinoline derivative of formula (IV) 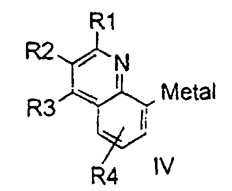 in which the meanings of R₁, R₂, R₃, R₄, R₅, B and n in the aldehyde of formula (VIII) and in the quinoline derivative of formula (IV) are those defined for the compound of formula (I) according to Claim 1 1 and Pg represents a protecting group, followed by a deprotection of the amine, to give a compound of formula (I), according to Claim 1 in which A represents a hydroxyl and R₆ represents a hydrogen, and optionally by a functionalization of the amine to give a compound of formula (I) according to Claim 1, in which R₆ is other than a hydrogen, and optionally followed by a dehydroxylation to give a compound of formula (I) in which A is a hydrogen.

7. Medicinal product, **characterized in that** it consists of a compound according to Claim 1, 2, 3, or 4.

8. Pharmaceutical composition, **characterized in that** it comprises at least one compound according to Claim 1, 2, 3, or 4 and one or more suitable excipients.
